Europäisches Patentamt

European Patent Office

Office européen des brevets

(19)

(11) **EP 1 710 287 A2**

(12) **EUROPEAN PATENT APPLICATION**

(43) Date of publication:
**11.10.2006 Bulletin 2006/41**

(51) Int Cl.:
*C09J 7/02* (2006.01)    *C08F 290/14* (2006.01)

(21) Application number: **06111750.3**

(22) Date of filing: **02.11.1999**

(84) Designated Contracting States:
**SE**

(30) Priority: **19.11.1998 EP 98203867**

(62) Document number(s) of the earlier application(s) in accordance with Art. 76 EPC:
**99962676.5 / 1 141 158**

(71) Applicant: **3M Innovative Properties Company St. Paul,
Minnesota 55133-3427 (US)**

(72) Inventors:
• **Von Jakusch, Egbert A.
D-42653, Solingen (DE)**

• **Jung, Dieter
D-47447, Moers (DE)**

(74) Representative: **Wilhelm, Stefan
3M Deutschland GmbH
Carl-Schurz-Strasse 1
41453 Neuss (DE)**

Remarks:
This application was filed on 27 - 03 - 2006 as a divisional application to the application mentioned under INID code 62.

(54) **Release coating compositionand method of making a release coated backing**

(57)     The present invention provides a method of making a release coated backing having a fibrous layer of woven fibers or of non-woven fibers of thermoplastic polymer, comprising the steps of coating a curable silicone release coating composition on the fibrous layer of the backing and curing the thus applied silicone release coating by exposing it to actinic radiation or heat, said curable silicone release coating composition comprising (i) a polydialkylsiloxane having acrylate and/or methacrylate groups and the ratio of the average number of dialkylsiloxane units to the average number of acrylate and methacrylate groups being between 10 and 15, and
(ii) an organic compound free of silicon and comprising at least two reactive groups selected from the group consisting of an acrylate and a methacrylate group, said organic compound free of silicon having a viscosity of at least 500 mPa·s at 25 °C,
and the weight ratio of said polydialkylsiloxane to said organic compound being between 8:92 and 35:65.

Fig. 2

EP 1 710 287 A2

**Description**

1. Field of the invention

The present invention relates to a method of making a release-coated backing that comprises a fibrous layer of woven fibers or of non-woven fibers of a thermoplastic polymer and that can be used in the manufacturing of adhesive tapes suitable for use in absorbent articles such as diapers to provide a mechanical closure tab for the absorbent article. In particular, the present invention relates to a silicone release coating that can be provided on the fibrous layer of the backing to provide easy unwinding of the adhesive tape from a roll.

2. Background of the invention

A disposable diaper typically has a thin, flexible, stretchy, low density polyethylene backsheet film, an absorbent core on the inside of the backsheet film, and a porous top sheet overlying the core. Such a diaper is positioned at the crotch of the wearer, the two ends of the diaper extending, respectively toward the front and back. Adjacent edges of the diaper at each side are then either positioned adjacent to each other or overlapped, a strip of pressure-sensitive adhesive tape or mechanical fastener tape being adhered to the back sheet at the border adjacent each of the two edges, holding the diaper closed.

A desirable closure system which is used for disposable articles employs a mechanical fastener, comprising for example, hook and loop fastening components. Mechanical fastening systems have the advantage that they may be repeatedly used for opening and refastening the disposable article. Closure systems which contain mechanical fasteners are described in US patents US 5,019,073 and US 5,176,671 and in European patent applications EP 0,324,578, EP 0,563,457 and EP 0,563,458.

A closure system employing a mechanical fastener typically uses an adhesive tape for attaching the mechanical fastener to the backsheet film at both edges of the disposable diaper. Up to now, an adhesive tape having a polymeric film backing has been used for attaching the mechanical fastener to the edges of the diaper. However, it would be desirable to replace the plastic film backing with a non-woven backing that creates a cloth-like feel. The latter is particularly desirable as many other parts of the diaper and in particular the outer surface thereof have a cloth-like feel.

Since the adhesive tape used for the manufacturing of the closure system is typically provided as a roll wherein the tape is rolled on itself, it is necessary to provide a release coating on the side of the backing opposite to the side containing the adhesive layer of the tape. However, adhesive layers used for attaching to the polyethylene surface of a diaper typically include an aggressive adhesive, for example one yielding a 90° peel adhesion of more than 6 N/2.54cm. Although release coatings for non-woven backings are known in the art, none of them was entirely satisfactory for use with an adhesive tape using an aggressive adhesive as is typically used in the production of closure systems for diapers. Problems encountered with the release coatings of the prior art include transfer of the release coating to the adhesive layer thereby affecting the adhesion performance thereof, difficult unwinding and damage to the non-woven backing of the tape upon unwinding.

Application of mechanical closures using the adhesive tape can be accomplished via in-line lamination of all fastening and release components on the diaper manufacturing line. However, in-line lamination complicates the manufacturing process of the desired products, and sometimes causes problems for the manufacturers. Accordingly, closure system fastening devices have been developed that are provided in roll form.

A single roll of closure tape in the form of a prelaminate contains all necessary elements directly in the line of manufacture. The closure tape is applied to the diaper as a composite tape, with the width of the roll being substantially the same as the desired length of the diaper closure to be fabricated. The closure tape is severed at right angles to the edges of the composite tape roll at intervals corresponding to the width of the desired closure tape and adhered at an appropriate location along the border of one side of the diaper. Since also in the case of such a closure tape in the form of a prelaminate, the adhesive layer contacts the other side of the backing when the tape is rolled, the same problems as those mentioned for the adhesive tape in roll form exist here as well.

US 4,696,854 discloses a porous bilayer non-woven substrate of an organic polymer layer and a cured silicone resin layer. An adhesive tape that can be wound upon itself can be produced by coating a hydrophilic natural or synthetic pressure sensitive adhesive on the organic polymer layer. It is further disclosed that such adhesive tapes are useful as medical types because the bilayer non-woven substrate has about the same porosity as the non-woven without the cured silicone resin layer. Since the adhesive tapes are intended for medical application, the preferred adhesive used is a pressure sensitive polyacrylic adhesive which are fairly unaggressive adhesives allowing painless peeling of the tape from the skin.

US 4,871,611 similarly relates to non-woven adhesive tapes intended for medical applications. This US-patent discloses coating of the non-woven substrate on one side with a radiation curable polysiloxane resin composition and curing the composition while avoiding substantial penetration into the non-woven backing. The exemplified coating compositions contain essentially 100 % of polysiloxane resin. The preferred silicone resin of US 4,871,611 includes (meth)acrylated polydialkylsiloxanes such as the TEGO™ Silicone Acrylates RC-149, RC-300, RC-450 and RC 802 available from Goldschmidt AG.

WO 88/7931 relates to release coatings that comprises 1 to 30 % by weight of a reactive silicone dispersed as a discontinuous phase in 99 to 70 % by weight of a reactive resin. Exemplified reactive silicones include polydimethylsiloxanes having an acrylic group, a mercapto group or an oxirane group. The reactive resin comprises reactive oligomers that have functional groups capable of reacting with the reactive silicone. The release coating can be provided on paper or polymeric film to produce for example labels.

WO 95/23694 relates to a silicone release coating that comprises a mixture of two different classes of (meth) acrylated silicones. Exemplified reactive silicones include the silicones available from Goldschmidt Chemical Corp. under the tradename TEGO™ RC. Particularly disclosed is TEGO™ RC-726 as an example of one class of silicones to be combined with RC-705, an example of the second class of silicones. The silicone release coating of this application may further optionally contain a reactive oligomer such as for example an acrylated or methacrylated polyhydroxy compound.

WO 96/5962 discloses radiation curable silicone release coatings that comprises a mixture of two different classes of (meth)acrylated silicones and a mono(meth)acrylate of a specific formula. Exemplified silicones include RC-726 and RC 708 both available from Goldschmidt Chemical Corp. and SL 5030 available from GE silicones. Reactive oligomers such a acrylated or methacrylated polyhydroxy compounds can also be added to the release composition. It is further taught that the release coating can be used on various substrates including paper, vinyl, PVC films, polyester films, polyolefin films, glass, steel, aluminum and non-woven fabrics.

US 5,562,992 discloses a radiation curable silicone release composition that comprises from 2 to 7 % of a (meth)acrylated silicone resin and from 90 to 98 % of an acrylated or methacrylated organic polyhydroxy compound or polyamino compound. Exemplified silicones include RC 450, RC 450N, RC 706, RC 707, RC 710, RC 720 and RC 726 all available from Goldschmidt Chemical Corp.. The release coating is said to be useful on various substrates including paper, vinyl, PVC films, polyester films, polyolefin films, glass, steel, aluminum and non-woven fabrics.

EP 0,693,889 discloses a loop fastener material including loops to be releasably engaged by mating fastener portions wherein the loop fastener material is provided as a roll or a stack. To control the adhesion between the loops of the loop layer and the adhesive layer when stored as a roll or stack, it is taught to coat the loops with a release agent such as a reactive silicone.

3. Summary of the invention

The present invention provides a method of making a release coated backing having a fibrous layer of woven fibers or of non-woven fibers of thermoplastic polymer, comprising the steps of coating a curable silicone release coating composition on the fibrous layer of the backing and curing the thus applied silicone release coating by exposing it to actinic radiation or heat, said curable silicone release coating composition comprising

(i) a polydialkylsiloxane having acrylate and/or methacrylate groups and the ratio of the average number of dialkylsiloxane units to the average number of acrylate and methacrylate groups being between 10 and 15, and
(ii) an organic compound free of silicon and comprising at least two reactive groups selected from the group consisting of an acrylate and a methacrylate group, said organic compound free of silicon having a viscosity of at least 500 mPa s at 25°C,

and the weight ratio of said polydialkylsiloxane to said organic compound being between 8:92 and 35:65.

The invention further relates to a release coating composition comprising:

(i) a polydialkylsiloxane having acrylate and/or methacrylate groups and the ratio of the average number of dialkylsiloxane units to the average number of acrylate and methacrylate groups being between 10 and 15,
(ii) an organic compound free of silicon and comprising at least two reactive groups selected from the group consisting of an acrylate and a methacrylate group, said organic compound free of silicon having a viscosity of at least 500 mPa s at 25°C, and the weight ratio of said polydialkylsiloxane to said organic compound being between 8:92 and 35:65, and
(iii) optionally a photo-initiator.

Finally, the invention provides a release coating obtained by curing a release coating comprising:

(i) a polydialkylsiloxane having acrylate and/or methacrylate groups and the ratio of the average number of dialkylsiloxane units to the average number of acrylate and methacrylate groups being between 10 and 15,
(ii) an organic compound free of silicon and comprising at least two reactive groups selected from the group consisting of an acrylate and a methacrylate group, said organic compound free of silicon having a viscosity of at least 500mPa s at 25°C, and the weight ratio of said polydialkylsiloxane to said organic compound being between 8:92 and 35:65, and

(iii) optionally a photo-initiator.

4. Brief description of the drawings

Fig. 1 is a perspective view of a diaper in a closed form;
Fig. 2 is a schematic representation of a cross-section of the closure tape tab of the invention;
Fig. 3 is a schematic representation of the patch comprising the mechanical fastener patch;
Fig. 4 is a cross-sectional view of preferred embodiments of the closure tape tab of the invention;
Fig. 5 is a cross-sectional view of the closure tape tab attached to one edge of a diaper, shown in the position during storage; and
Fig. 6 is a perspective view of a roll of the closure tape.

5. Detailed description of the invention

The backing of the adhesive tape of the present invention comprises a fibrous layer of woven fibers or of non-woven fibers of a thermoplastic polymer. Examples of thermoplastic polymers include polyolefins such as polyethylene and polypropylene and copolymers thereof, polyesters such as polyethyleneterephthalate and polyamides such as NYLON 6 or NYLON 66. The fibrous layer may be formed as a woven or non-woven web by any technique conventionally used in the art.

Woven webs include those made by processes which produce regular, repeating and mechanically entangled arrangements of fibers.

Non-woven webs of fibers of thermoplastic polymer include non-woven webs manufactured by any of the commonly known processes for producing nonwoven webs including dry laid (carded or air-laid) webs, wet laid webs and polymer laid webs. The latter is preferred for forming the fibrous layer of the backing and can be made by spunbonding techniques or melt-blowing techniques or combinations of the two. Spunbonded fibers are typically small diameter fibers which are formed by extruding molten thermoplastic polymer as filaments from a plurality of fine, usually circular capillaries of a spinnerette with the diameter of the extruded fibers being rapidly reduced. Spunbond fibers are generally continuous and typically have an average diameter of at least 7 $\mu$m, preferably 15 $\mu$m to 30 $\mu$m. Meltblown fibers are typically formed by extruding the molten thermoplastic material through a plurality of fine, usually circular, die capillaries as molten threads or filaments into a high velocity, usually heated gas (e.g. air) stream which attenuates the filaments of molten thermoplastic material to reduce their diameter. Thereafter, the meltblown fibers are carried by the high velocity gas stream and are deposited on a collecting surface to form a web of randomly disbursed meltblown fibers. The average diameter of the meltblown fibers is preferably less than 10 $\mu$m. Any of the non-woven webs may be made from a single type of fiber or two or more fibers which differ in the type of thermoplastic polymer and/or or thickness.

In connection with a particular embodiment of the present invention, the backing may comprise multiple layers of fibrous layers that may vary in the type of fibers used and/or the particular technique used to produce the fibrous layer. For example, in connection with a particularly preferred embodiment, the backing comprises a fibrous layer formed by spunbonded fibers and a fibrous layer formed by melt-blown fibers. According to a still further embodiment, the backing may comprise in the order given a fibrous layer of spunbonded fibers, one or two layers of meltblown fibers and a fibrous layer of spunbonded fibers. The advantage of using a backing that comprises a combination of a spunbonded fibrous layer and a meltblown fibrous layer is that the meltblown fibrous layer may function as a barrier against absorption of the adhesive coating, in particular when the latter has low viscosity.

Fibers of the non-woven webs can be bonded to each other to provide improved web cohesion by any of the known techniques, including thermal bonding using an oven or a calender roll, which can in turn be either smooth or embossed. Bonding may also be accomplished by needle punching or stitch bonding. Hydroentangling methods may also be employed. The level of bonding generally determines the porosity of the fibrous layer.

The fibrous layers typically have a basis weight of between 5 g/m$^2$ and 1000 g/m$^2$, preferably 10 to 100 g/m$^2$ and most preferably 20 to 60 g/m$^2$. Especially non-woven webs are defined preferably by basis weight rather than thickness which is highly dependent on measurement technique. The fibers of the fibrous layer typically will have a diameter of 5 $\mu$m to 35 $\mu$m. While the silicone release coating of this invention is particularly suitable for providing a release coating on these fine fiber based layers, the silicone release coating is also suitable for coating layers based on thicker fibers.

According to a further embodiment of the present invention, the backing of the adhesive tape may comprise a laminate of the fibrous layer and a polymeric film layer. Suitable polymeric film layers include polyester film, polypropylene film, polyethylene film and other polyolefin films. The polymeric film layer will typically have a thickness of 10 μm to 200 μm and may be advantageous to avoid penetration of the adhesive coating into the fibrous layer.

Instead of providing a polymeric film layer as a barrier against penetration by the adhesive coating into the fibrous layer, it is furthermore possible to calender the fibrous layer with a hot roll so as the reduce the porosity of the fibrous layer on one side thereof.

In accordance with the present invention, the fibrous layer of the backing is provided with a silicone release layer. The silicone release layer comprises the cured reaction product of a polydialkylsiloxane having acrylate and/or methacrylate groups and an organic compound free of silicon and comprising at least two reactive groups selected from the group consisting of an acrylate and a methacrylate. Preferred polydialkylsiloxanes include those that have a ratio of the average number of dialkylsiloxane units to the average number of acrylate or methacrylate groups between 10 and 15. Particularly preferred for use in this invention are polydimethylsiloxanes having a ratio of the average number of dimethylsiloxane units to the average number of acrylate or methacrylate groups between 10 and 15. Suitable polydialkylsiloxanes for use in this invention typically have a viscosity in the range of 100 to 500 mPa s. Examples of suitable silicones for use in this invention are commercially available and include TEGO™ RC-902 and RC-715 both available from Goldschmidt Chemical Corp.

The organic compound free of silicon for reaction with the polydialkylsiloxane is typically an acrylated or methacrylated polyhydroxy compound or an acrylated or methacrylated polyamino compound. The organic compound has at least two and more preferably at least three acrylate and/or methacrylate groups. The organic compound should preferably also have a viscosity of at least 500 mPa·s at 25 °C and more preferably at least 800 mPa·s at 25 °C. Particular examples of organic compounds for use in the invention include ditrimethylol propane tetraacrylate available as E 140 from UCB Chemicals, Belgium and E 810, a polyester tetraacrylate available from UCB. Modified pentaerythritol triacrylate (viscosity ca. 500 mPa·s) available as SR-444 from Sartomer, aliphatic diacrylate oligomer (viscosity ca. 1000 mPas) availabe as CN-132 from Sartomer, ethoxylated bisphenol A dimethacrylate (viscosity ca. 1100 mPa·s) available as SR-348 from Sartomer, bisphenol A derivative diacrylate oligomer (viscosity ca. 1400 mPa·s) available as E-150 from UCB.

On the side of the backing opposite to the side containing the silicone release layer, there is provided a pressure sensitive adhesive layer. Suitable pressure sensitive adhesives for use in the adhesive layer of the adhesive tape are the pressure sensitive adhesives commonly used in adhesive tapes for diapers. The pressure sensitive adhesive should be capable of providing an adhesive tape having a 90° peel adhesion of at least 6 N/2.54cm. This 90° peel adhesion is the adhesion obtained after the adhesive layer has been in contact with the silicone release layer, i.e. the adhesive tape should retain a 90° peel adhesion of at least 6 N/2.54cm after being unwound from a roll. Preferably, the pressure sensitive adhesive layer of this invention comprises a rubber based adhesive of which the tackiness has been modified by the inclusion of a tackifying resin. The rubber resin preferably comprises an A-B-A block copolymer wherein the A blocks are derived from a styrene monomer and the B blocks are derived from isoprene, butadiene or hydrogenated version of these.

Such adhesives are disclosed generally in U.S. Pat. Nos. 3,419,585, 3,676,202, 3,723,170 and 3,787,531. U.S. Pat. Nos. 3,676,202 and 3,723,170 disclose the advantages of these adhesives when the B-blocks are derived from isoprene and the A-blocks preferably are derived from styrene, including the advantages of particular tackifiers. U.S. Pat. Nos. 3,519,585 and 3,787,531 disclose the advantages of mixing certain A-B block copolymers with certain A-B-A block copolymers in similar pressure-sensitive adhesive formulations.

A particularly preferred adhesive composition is disclosed in US 4,136,071 and comprises a thermoplastic elastomeric component and a resin component and the thermoplastic elastomeric component consists essentially of about 50-90 parts, preferably about 60-80 parts, of a linear or radial styrene-isoprene-styrene A-B-A block copolymer and about 10-50 parts, preferably about 20-40 parts of a simple styrene-isoprene A-B block copolymer. The A-blocks are derived from styrene or styrene homologues and the B-blocks are derived from isoprene, either alone or in conjunction with small proportions of other monomers, in both the A-B-A and A-B block copolymers.

As indicated above, the A-B-A block copolymers preferably are of the type which consists of A-blocks (end blocks) derived, i.e., polymerized, from styrene or styrene homologues; and B-blocks (center blocks) derived from isoprene either alone or in conjunction with small proportions of other monomers. The individual A-blocks preferably have a number average molecular weight of at least about 7,000, preferably in the range of about 12,000 - 30,000, and the A-blocks preferably constitute about 10 - 35 percent by weight of the block copolymer. The number average molecular weight of the B-blocks for linear A-B-A block copolymers preferably is in the range of about 45,000 - 180,000 and that of the liner copolymer, itself, preferably is in the range of about 75,000 - 200,000. The number average molecular weight of the radial A-B-A block copolymers preferably is in the range of about 125,000 - 400,000, and that of the corresponding B-blocks preferably is about 95,000 - 360,000.

Useful radial A-B-A polymers are for example of the type described in U.S. Pat. No. 3,281,383 and conform to the following general formula: (A-B-)(n) X, wherein A is a thermoplastic block polymerized from styrene or styrene homologues, B is an elastomeric block polymerized from a conjugated diene such as butadiene or isoprene, X is an organic or inorganic connecting molecule, with a functionality of 2-4 as described in U.S. Pat. No. 3,281,383 or possibly with a higher functionality as described in the article entitled "New Rubber is Backed by Stars" appearing on page 35 of the June 11, 1975 issue of Chemical Week. "n" then is a number corresponding to the functionality of X.

In the styrene-isoprene derived A-B block copolymers, the number average molecular weight of the individual A-blocks is typically about 7,000 - 20,000 and the total molecular weight of the block copolymer generally should not exceed about 150,000. A-B block copolymers based on styrene and isoprene are described generally in U.S. Pat. No. 3,787,531.

The elastomeric component of the preferred adhesive composition may include small amounts of other more conventional elastomers but these should not exceed about 25 percent by weight of the elastomeric component. These include natural rubbers, synthetic rubbers based on butadiene, isoprene, butadiene-styrene, butadiene-acrylonitrile and the like, butyl rubbers, and other elastomers.

The preferred adhesive composition includes about 20 - 300 parts preferably 50 - 150 parts, of resin component, per one hundred parts by weight of the thermoplastic elastomeric component. The resin component consists essentially of tackifier resins for the elastomeric component. In general any compatible conventional tackifier resin or mixture of such resins may be employed. These include hydrocarbon resins, rosin and rosin derivatives, polyterpenes, and other tackifiers.

The adhesive layer may also include small amounts of various other materials such as anti-oxidants, heat stabilizers and ultraviolet absorbers, fillers, and the like. Typical antioxidants are 2,5 ditertiary amyl hydroquinone and ditertiary butyl cresol. Similarly, conventional heat stabilizers such as the zinc salts of alkyl dithiocarbamates may be used. Similarly, the particulate mixture of this invention may include small amounts of fillers and pigments such as zinc oxide, aluminum hydrate, clay, calcium carbonate, titanium dioxide, carbon black and others.

The adhesive layer is typically provided at a thickness of 20 to 200 $\mu$m and more preferably at a thickness of 25 $\mu$m to 100 $\mu$m.

The adhesive tape of the present invention can be produced by coating a curable silicone release coating composition on the fibrous layer of the backing and curing the thus applied silicone release coating by exposing it to actinic radiation or heat. The curable silicone release coating composition comprises (i) a polydialkylsiloxane having acrylate and/or methacrylate groups and the ratio of the average number of dialkylsiloxane units to the average number of acrylate and methacrylate groups being between 10 and 15, and (ii) an organic compound free of silicon and comprising at least two reactive groups selected from the group consisting of an acrylate and a methacrylate group. The organic compound free of silicon should preferably have a viscosity of at least 500 mPa·s at 25 °C, and the weight ratio of the polydialkylsiloxane to the organic compound is generally between 8:92 and 35:65, preferably between 10:90 and 30:70.

The silicone release coating composition can be applied to the fibrous layer of the backing by any conventional means known in the coating art. Particular examples of suitable coating techniques include, roller coating, brushing, spraying, reverse roll coating, gravure coating and die coating. In a particularly preferred embodiment of this invention, the silicone release coating composition is substantially free of solvents and is applied by multi roll coating. By the term substantially free of solvents is meant that the coating composition contains less than 10 % by weight and more preferably less than 5 % by weight of solvents.

Curing of the coated silicone release composition can be effected by heat or photochemically by irradiation with actinic radiation such as for example electronic beam, UV light, x-ray, gamma-ray and beta ray. In a preferred embodiment of the present invention, the silicone release coating composition also comprises a thermal or photochemical free radical initiator. Examples of useful photochemical free radical initiators, i.e. photoinitiators, which may be used in combination with ultraviolet light include, for example, benzyl ketals, benzoin ethers, acetophenone derivatives, ketoxime ethers, benzophenone, benzo or thioxanthones, etc. Specific examples of photoinitiators include: 2,2-diethoxyacetophenone; 2- or 3-or 4-bromoacetophenone; benzoin; benzophenone; 4-chlorobenzophenone; 4-phenylbenzophenone; benzoquinone; 1-chloroanthroquinone; p-diacetyl-benzene; 9,10-dibromoanthracene; 1,3-dephenyl-2-propanone; 1,4-naphthyl-phenylketone; 2,3-pentenedione;propiophenone; chlorothioxanthone; xanthone; fluorenone; and mixtures thereof. An example of a commercially available photoinitiator of this type is available from the Ciba Geigy Corporation of Hawthorne, N.Y. under the trade name of Darocur 1173. An alternative photoinitiator is a mixture of 70% oligo 2-hydroxy-2-methyl-1-4-(1-methylvinyl) phenyl propanone and 30% 2-hydroxy-2-methyl-1-phenyl-1-propanone available as ESACURE KIP 100F from Lamberti spa (Albizzate, Italy).

When present, the free radical initiator is typically used in an amount of 1 to 10 parts by weight for 100 parts by weight of silicone and more preferably in an amount of 2 to 5 parts by weight for 100 parts by weight of silicone.

The radiation-curable compositions of the present invention can be stabilized against premature polymerization during storage by the addition of conventional polymerization inhibitors such as hydroquinone, monomethylether of hydroquinone, phenothiazine, di-t-butyl paracresol, etc. Amounts of 0.1 weight percent or less of the stabilizers are generally effective.

To obtain an adhesive tape of the present invention, the side of the backing opposite to the side having the silicone release coating composition is provided with an adhesive layer. So as to avoid that the adhesive upon coating would penetrate through the backing, the backing is preferably provided with a barrier to avoid such penetration. Suitable barrier means have been described above and include a laminated polymeric film, a backing that comprises a combination of spunbonded and meltblown fibrous layers and treating one side of the fibrous layer with a hot calender roll. The adhesive layer can be provided on the backing by means any of the coating techniques known in the art or the adhesive layer can be first coated to a release liner and then transfer laminated on the backing. Suitable coating techniques to apply the adhesive layer onto the backing include hot melt coating techniques as well as solvent or water borne coating techniques such as knife coating, die coating, spraying, curtain coating and flat bed coating.

After the adhesive layer has been provided on the backing, the adhesive tape can be wound on itself to provide the adhesive tape in roll form.

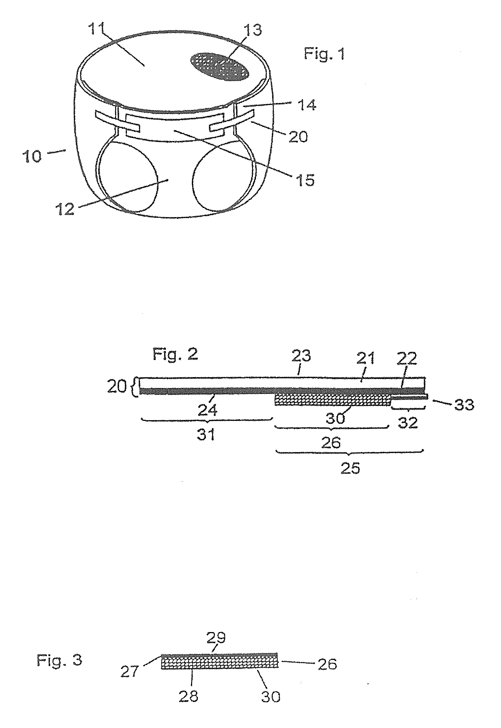

The adhesive tape of the present invention is intended for the manufacture of a closure system on an absorbent article and in particular a closure system on a diaper. Fig. 1 is a perspective view of a disposable diaper 10 in a closed form. The diaper comprises an absorbent core 13 between an inside surface 11 and an outside surface 12. The absorbent core 13 may be any means which is generally compressible, conformable, non-irritating to the wearer's skin, and capable of absorbing and retaining liquids and certain body exudates.

The outside surface 12 of the diaper is impervious to liquids and is preferably manufactured from a thin plastic film, although other flexible, liquid-impervious materials may also be used. The outside surface 12 prevents the exudates absorbed and contained in the absorbent core, from soiling articles which contact the diaper 1 such as bed sheets and undergarments.

The inside surface 11 of the diaper is compliant, soft-feeling, and non-irritating to the wearer's skin. Further, the inside surface 11 is liquid-pervious, permitting liquids to readily penetrate through its thickness. A suitable inside surface 11 may be manufactured from a wide range of materials such as porous foams, reticulated foams, apertured films, natural fibers (e.g. wood or cotton fibers), synthetic fibers (e.g. polyester or polypropylene fibers) or from a combination of natural and synthetic fibers. Preferably, it is made of a hydrophobic material to isolate the wearer's skin from liquids retained in the absorbent core 13. A suitable inside surface 11 may be, for example, a spun-bond or carded polypropylene non-woven of approximately 15-25 g/m$^2$.

The absorbent core 13 may be secured to the outside surface 12 by means of, for example, pressure-sensitive adhesives, hot melt adhesives or other adhesives, ultrasonic bonding or heat/pressure sealing. The outside surface 12 and the inside surface 11 may be joined to each other directly or indirectly by using an intermediate fixing member to which the outside surface 12 and the inside surface are affixed. The inside surface 11 and the outside surface 12 may be associated together by various means comprising, for example, pressure-sensitive adhesives, hot melt adhesives or other adhesives, ultrasonic bonding and/or heat and/or pressure.

The above description of the diaper 10 is meant to be explanatory only and not limiting. Further details on diapers and their construction are described in literature and may be taken, for example, from EP 0,529,681, US 4,036,233, EP 0,487,758, WO 96/10382, US 3,800,796, EP 0,247,855 or US 4,857,067.

The adhesive tape of the invention can be used in the in-line lamination of all fastening and release components on the diaper manufacturing line. However, it is often more convenient for a manufacture to use a single roll of closure tape that is in the form of a prelaminate having all necessary elements to manufacture the closure system.

Accordingly, in connection with a particular embodiment of the present invention there is also provided a prelaminated composite tape from which composite adhesive closure tabs can be cut to produce a closure system on an absorbent article, in particular a diaper. The typical components of such a prelaminated composite tape have been described in for example WO 96/21413.

As shown in Fig. 2, composite adhesive closure tab 20 has a backing 21 that comprises the fibrous layer. Adhesive layer 24 is applied to the fastening surface 22 of backing 21. The silicone release layer of the invention is provided on the back side surface 23 of the backing 21.

The backing 21 has a fastening surface 22 which is provided with the adhesive layer 24. To the first axial extending section 25 of the backing 21, a patch 26, comprising a mechanical fastener component 30, is disposed on the adhesive layer 24. The second axial extending section 31 of the backing 21 will be attached permanently to the edge portion 14 of the disposable diaper or garment 10 in the manufacturing process by the adhesive layer 24.

The general structure of the mechanical fastener patch 26 is shown in Fig. 3. It is essentially composed of a base sheet 27 with a first surface 29 which may be attached to the backing 21 by the adhesive layer 24 and a second surface 28 on which a mechanical fastener component 30 is provided. The base sheet 27 and the mechanical fastener component 30 may consist of the same or of different materials.

The mechanical fastener component 30 may be a part of any conventional mechanical fastener systems. A suitable fastening system is a hook and loop fastener comprising two interlocking materials, one of the components is a hook material and the other is a loop material. One component of the mechanical fastening system is part of the patch 26, and the other is on the target area 15 on the outside surface 12 of the disposable diaper or garment 10. For example, the target area 15 may be a strip which is attached to the outside surface 12 of the diaper 10 in such a manner that the size of the diaper or garment may be adjusted in accordance with the size of the user. The target area can comprise one or more stripes and could form the entire outside surface 12 of the diaper. Depending on the material of the outside surface 12, it may be possible that the fastening system will be releasably attached directly to the outside surface 12. For example, in case the mechanical fastener component 30 comprises a hook material, such hook material may be attached to a material being comprised of woven or non-woven fabric or any other suitable material which interlocks with a hook. The closure system comprises composite adhesive closure tab 20 with the mechanical fastener patch 26, together with target area 15.

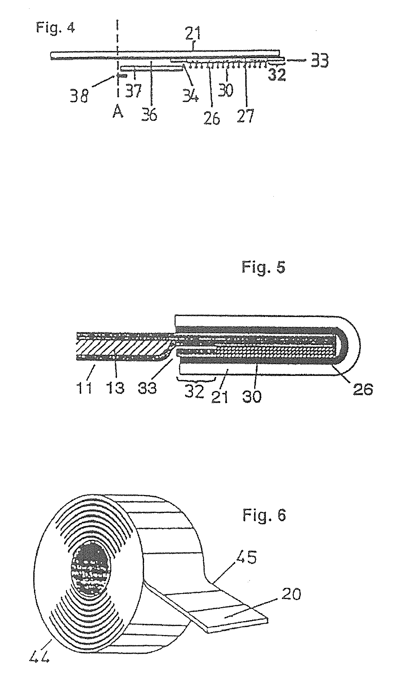

In Fig. 4 an embodiment is shown wherein a spacer 34 is arranged beside the patch 26 on the first extending section 25 to provide a non-adhesive region. The spacer 34 may be used in order to make the disposable article more comfortable for the wearer. The spacer may be formed in various manners, such as providing a region of the backing 21 with no adhesive layer 24. Alternatively, a spacer material 34 such as a polymeric film material, paper or non-woven material may be attached to the adhesive layer 24. In further embodiments the area 34 can be rendered non-adhesive by designated contamination for example with grease, talcum or similar.

In order to increase the fastening strength of the closure tape, it may be possible to use two or more different mechanical fastener components. For example, two different hook materials could be used, the hooks of one component oriented in a direction opposite to the orientation of the hooks of the second component.

As is further shown in Fig. 4, the composite adhesive closure tab comprises an adhesive section 36 that facilitates the disposal of the disposable article 10 after use. Further, the composite adhesive closure tab 20 may be easily folded inside and maintained in the folded position during storage as shown in Fig. 5.

The adhesive section 36 is formed by uncovered adhesive layer 24 between the patch 26 comprising the mechanical fastener component 30 and the edge portion 14. A release tape 37 is disposed on the adhesive section 36. The release tape 37 covers the adhesive section 36 and extends from the edge portion 14 to the non-adhesive area 34. In case the release tape 37 covers also the non-adhesive area 34, it may be easily removed. The other end of the release tape is provided on the edge portion 14 of the diaper 10. The release tape 37 protects the adhesive section 36 from contamination during storage and transport. As a release tape 37 any suitable release tape composed of three layers, i.e. of a support sheet comprising an adhesive layer and a non-adhesive layer, may be used. For transport and storage the closure tape is folded along the folding line A and the release tape 37 adheres to the inside surface 11. When opening the folded closure tape for use, the release tape 37 remains on the inside surface 11. During use, the open adhesive of the section 36 adheres to the target area 15 and supports the mechanical fastening system. After use, the diaper 10 can be folded or rolled into a structure, such that the closure tape tab still extends outwardly from the rolled-up disposable article 10. The adhesive section 36 is then secured to either the target area 15 or to the backsheet outside surface of the backsheet 12 so as to secure the disposable article 10 in its rolled-up configuration such that it may be easily and conveniently disposed in a waste receptacle.

For distribution of forces during use of the diaper, a center strip 38 may be disposed on the line where the release tape 37 adjoins the edge 14 of the diaper.

As shown in Fig. 4, the composite adhesive closure tab comprises a finger tab 33. In one possible embodiment of a finger tab, the material used to make a finger tab 33 will be a thin film, for example polypropylene film, non-woven, paper. The thin film is attached to the fastening surface 22 at the free end 32 of the first axial extending section 25 of the backing 21. In an alternative embodiment the free end 32 of the support sheet 21 is free form adhesive layer 24. In a third alternative embodiment the free end 32 of the support sheet 21 is completely coated with the adhesive layer 24 and then folded over.

The width of the roll of the prelaminated composite depends on the intended application. Usually, the rolls of prelaminated composite tape for disposable articles, have a width of about 30 to about 100 mm and preferably from about 50 to about 70 mm. In order to provide a closure tape system which can be wound up to a stable roll, the first axial extending section 25 occupies 55 to 70 %, and the second axial extending section 31, 30 to 45 %, of the width of the strip. In case a finger lift is used, the finger lift occupies 8 to 12%, the extending section 25 occupies 46 to 62 %, and the second axial extending 31 occupies 30 to 42 %, of the total width of the strip.

The composite adhesive closure tab 20 can be cut from a stock roll 44. In use, a segment of a roll 44 of prelaminated composite tape 45 is cut from the roll in a desired length; see Fig. 6, where the line shows where the tape is cut into tabs. Thereupon the fastening surface 22 of the backing 21 is secured to the outside surface 12 of the edge portion 14 of the diaper or garment 10. The first axial extending section 25 is folded around the edge portion 14 and the closure tape is positioned in a folded form as shown in Fig. 5. Now, it is in the pre-use position.

The closure tape is folded so that it will not be unfolded prior to use or during manufacturing and storage. It is contemplated that a diaper would be sold to the consumer in this condition.

In use, a diaper 10 containing the composite adhesive closure tab 20 of the present invention is positioned around a wearer. To fit the diaper 10, the folded composite adhesive closure tab 20 is unfolded, for example the finger tab 33 is grasped and the tape 20 is then unfolded. The extending section 25 comprising the mechanical fastener component 30 is then secured to the outside surface 12, preferably to the target area 15. When the same process is followed on the other side of the wearer, the diaper is then secured in place.

The invention is further illustrated by means of the following drawings without however the intention to limit the invention thereto.

Test methods

*Measurement of amount* of *release composition on the surface* of a *fibrous layer*

The amount of release composition present on the surface of the fibrous layer was measured using an X-ray fluorescence analyser Model 200TL available from ASOMA Instruments, Austin, Texas. The test method is available from Th. Goldschmidt AG, Essen, Germany, in two parts: a calibration technique available as KM RC 005 A and a measurement technique available as SM 316 A.

The calibration technique involves first coating and curing known quantities of UV curable polydimethysiloxane (PDMS)(available as TEGO-RC 726 from Th. Goldschmidt) on a polyester film. At least five differing known coating weights were coated, UV-cured and measured for their x-ray fluorescence. A calibration curve relating x-ray fluorescence to amount of PDMS present was then prepared.

The measurement technique employs x-ray fluorescence of silicon atoms. A test substrate having some unknown amount of silicone on its surface is subjected to x-ray fluorescence. The absolute value of the fluorescence is measured and the corresponding amount of PDMS is read from the calibration curve.

The actual amount of release composition on the surface was then calculated from the amount of measured PDMS using the following formula:

$$\text{Release Composition on Surface in } g/m^2 =$$

$$(g\ PDMS/m^2 \text{ measured on coated substrate - } g\ PDMS/m^2 \text{ measure on uncoated substrate) / Factor for the release composition}$$

The elements to be fed into the formula are determined by the following:

1. g $PDMS/m^2$ measured on coated substrate is read from the calibration curve relating amount of fluorescence to amount of PDMS present

2. g $PDMS/m^2$ on uncoated substrate is that value measured for the fibrous web with no release composition (corresponding to a "blank" measurement)

3. A factor is given by Th. Goldschmidt in the test method (an "RC number") for each of the UV-curable (meth) acrylated silicones. This is essentially the fraction of the UV-curable silicone which is PDMS. The factor is always less than 1.0 and is lower for UV-curable silicones which have less silicone content (more acrylate groups). The factor for the release composition is calculated in a weighted average fashion using the amount and factor for each of the materials employed. Additives which have no polydimethyl siloxane units (free of silicon atoms) are given a factor of zero.

*90° peel adhesion*

Peel adhesion was measured using a portion of tape unwound from a roll of tape as prepared in the examples. The test evaluated the ability of the adhesive tape to adhere to a polyethylene surface after the adhesive had been in contact with the silicone-based release material.

The polyethylene film used in this test was prepared as follows. A low density polyethylene resin (Tenite 1550P available from Eastman Chemical Co., Kingsport, Tennessee, USA) having a melt index of 3.5 grams/10 min and a density of 0.918 grams/cm$^3$ was extruded at a melt temperature of 182 °C vertically downward through a conventional coat-hanger slot extrusion die. The melt exiting die was drawn into a nip formed by a mirror finish crome roll (8 °C inlet water temperature) and a silicone rubber roll (7 °C inlet water temperature) resulting in a film of 330 microns thickness. The surface of the film contacting the chrome roll was used for test purposes. The surface of the film used for testing the peel adhesion had an average surface roughness value $R_a$ of 1.4 $\mu$m and an average peak to valley height value $R_z$ of 12.5 $\mu$m. The roughness values $R_a$ and $R_z$ were determined by a laser profilometer available from UBM Messtechnik GmbH, Ettlingen, Germany, model number UB-16. The roughness values were calculated by this machine in accordance with Deutsche Industrie Norm (DIN) 4768 and DIN 4762.

The substrate for the test was prepared by first firmly bonding a 30 micron thick microembossed polyethylene film commonly used as a diaper backing to the stainless steel test plate surface. The polyethylene film was adhered to the test plate with double-coated adhesive tape (available as Tape 410 from 3M Company).

90° peel adhesion was measured using a tensile tester adapted in a special configuration to allow a peel angle of 90° to be maintained during the test. The equipment configuration is described in FINAT TEST METHOD NO. 2, a standard 90° peel test method (available from Federation Internationale des Fabricants Europeens et Transformateurs d' Adhesifs et Thermocollants sur Papiers et autres Supports (FINAT)).

There were several exceptions to the described FINAT method which are given below:

1) FINAT 2 requires a glass substrate which has been replaced by polyethylene film
2) a "standard FINAT test roller" was replaced by a 2 kg roller
3) the sample was rolled down once in each direction at 300 mm/min rather than twice at 200 mm/min as called for by the FINAT method
4) the dwell time was essentially zero rather than 20 min and 24 hrs, respectively, as required by the FINAT method.

A sample of test tape 30 cm long and 2.54 cm wide was adhered to the polyethylene surface of the test plate by rolling it down two times with a 2 kg roller. A portion of the tape end was left free for grasping in the test apparatus as described in the test method. After a dwell time of less than one minute, the test tape was peeled from the polyethylene substrate at a speed of 300 mm/min.

90° peel was recorded in units of N/2.54 cm .Three samples were evaluated and the results averaged.

*Roll unwind force (initial and aged)*

Unwind force was measured according to Association Des Fabricants Europeens De Rubans Auto-Adhesifs (AFERA) 4013.

A roll of adhesive tape prepared according to the Examples and having a length of ca. 30 m and a width 5 cm was evaluated using a modified tensile tester. The unwind rate was 500 m/min. The unwind force was recorded in cN/2.54 cm. Three measurements were made and the results averaged.

Unwind force was measured on rolls of tape which had been prepared and stored at ambient conditions for 24 hours as well as rolls of tape which were stored in a forced air oven at 50 °C for 15 days.

*90° release (modified Keil test)*

This test represents a second method (in addition to the Roll Unwind) for measuring the force required to separate an adhesive tape from a release-coated surface.

A test tape 5 cm wide and 10 cm long adhered to a steel substrate by placing the adhesive surface on the plate and rolling over twice with a 2 kg roller.

A test tape 2.54 cm wide and 10 cm long was then placed on top of the test tape in a manner such that the adhesive layer of the second test tape contacted the release coating of the first substrate tape. The test tape was rolled down two times with a 2 kg roller.

The tape laminate was then placed in a forced air oven at 70 °C and a 450 g weight was then allowed to rest on top of the bonded tape laminate for 20 hours while it was heated to 70 °C.

The test laminate was then removed from the oven and allowed to rest at 23 °C and 50 % relative humidity for 24 hours before testing.

The adhesive of the second tape was peeled from the backing of the first tape using the 90° peel adhesion method described under 90° Peel Adhesion Test above at a peel speed of 300 mm/min.

Each material was evaluated three times and the results averaged. Data was recorded in cN/2.54 cm.

Materials utilized in the Examples and Comparative Examples

*(Meth)acrylate-functional silicones*

RC 706 is an acrylate-functionalized polydimethylsiloxane having a functionality of 8:1 (8.0) available as TEGO RC-706 from Th. Goldschmidt AG, Essen Germany RC 708 is a 70/30 weight:weight mixture of an acrylate-functionalized polydimethylsiloxane (RC-726, functionality 108:6 or 18) and MQ resin, a functional silicone resin known to increase release forces. RC 708 available as TEGO RC-708 from Th. Goldschmidt AG, Essen Germany

RC 711 is an acrylate-functionalized polydimethylsiloxane having a functionality of 10:4 (2.5) available as TEGO RC-711 from Th. Goldschmidt AG, Essen Germany

RC 715 is an acrylate-functionalized polydimethylsiloxane having a functionality of 26:2 (13.0) available as TEGO RC-715 from Th. Goldschmidt AG, Essen Germany

RC 726 is an acrylate-functionalized polydimethylsiloxane having a functionality of 108:6 (18.0) available as TEGO RC-726 from Th. Goldschmidt AG, Essen Germany

RC-902 is an acrylate-functionalized polydimethylsiloxane having a functionality of 56:4 (14.0) available as TEGO RC-902 from Th. Goldschmidt AG, Essen Germany

"Functionality" is defined as the ratio of number of dimethyl siloxane units / number of (meth)acrylate groups.

*Organic compounds*

E 140 Ditrimethylol propane tetraacrylate (DMPTA), $M_w$ = 438, viscosity = 1100 mPa s, available as E 140 from UCB Chemicals, Drogenbos, Belgium

E 810 Polyester tetraacrylate $M_w$ ca. 1000, viscosity = 500 mPa s, available as E 810 from UCB Chemicals, Drogenbos, Belgium

SR 610 Polyethylene glycol 600 diacrylate, viscosity ca. 50-100 mPa s, available as SR 610 from Cray Valley Sartomer, Paris, France.

HDDA Hexanedioldiacrylate, viscosity ca. 10 mPa s

TPGDA Tripropyleneglycol diacrylate, viscosity ca. 15 mPa s

*UV Polymerization Initiator*

DAROCUR 1173 2-Hydroxy-2-methyl-1-phenyl-propan-1-one UV Photo-initiator available from from Ciba-Geigy, Basel, Switzerland.

Examples

*Example 1*

A backing was prepared by first heat embossing a 50 g/m$^2$ spun bond polypropylene non-woven web (fiber thickness ca. 20 microns, available as DALTEX 1050 from Don & Low, Angus, Scotland) with a thermally bonded pattern of diamond-shaped spots having a width of ca. 6 mm. The total embossed area was 19 %. The embossed non-woven was then heat laminated to a 30g/m$^2$ film comprising a polyolefin blend (75% polypropylene available as PPC 5660 from FINA and 25% linear low density polyethylene (LDPE) available as LE 7520 from Borealis, Lyngby , Denmark).

A mixture of 30 wt % UV-curable silicone (available as TEGO RC-715 from Th. Goldschmidt AG, Essen Germany), 70 wt % ditrimethylolpropanetetraacrylate (DMPTA, $M_w$ = 438, viscosity = 1100 mPa s, available as E140 from UCB Chemicals) and 3 wt. % UV polymerization initiator (available as DAROCUR 1173 from Ciba-Geigy, Basel, Switzerland) was prepared by mixing the three components using a motor driven propeller mixer for 10 min at a temperature of 23 °C to form a homogeneous, milky white suspension. The suspension was then applied to the non-woven surface of the backing in an amount of 2.5 g/m$^2$ using a multi-roll coater.

The coating was then cured in inert atmosphere (nitrogen) using UV light by passing the coated non-woven web under medium pressure 120 W/cm mercury lamps (available as MODEL F-450 from Fusion UV Systems, Inc., Gaithersberg, Maryland). The distance from lamp to the surface of the backing was 2.5 cm.

A single layer of pressure-sensitive adhesive was then applied to the opposite, film-bearing side of the backing. The adhesive was based on a mixture of synthetic block copolymer and tackifying resin and was applied by conventional hot-melt coating techniques using a drop die in an amount of 35 g/m$^2$.

The adhesive tape thus prepared was wound into a roll and then slit into thinner rolls of 2.54 cm in width. The components used to make the adhesive tape are summarized in Table 1.

The roll thus prepared were unwound and tested for adhesion and shear to polyethylene. Roll unwind forces were measured. A Keil test was also used to measure the release force between the adhesive layer and the silicone-coated fibrous layer

Adhesive tape behavior and properties are summarized in Table 2.

*Examples 2 and 3*

Example 1 was repeated with the exception that the amount of silicone in the release composition was reduced to 20 % and 10 %, respectively.

*Example 4*

Example 1 was repeated, with the exception that RC-902 (functionality = 14, viscosity = ca. 350 mPa·s) was substituted for RC 715.

*Example 5*

Example 2 was repeated with the exception that the organic compound employed was a polyester tetraacrylate, $M_w$ ca. 1000, viscosity = 500 mPa·s, available as E 810 from UCB Chemicals, Drogenbos, Belgium.

*Example 6*

Example 6 employed the same release coating composition as in Example 1 (a mixture of 30 wt % UV-curable silicone (available as TEGO RC-715 from Th.

Goldschmidt AG, Essen Germany), 70 wt % ditrimethylolpropanetetraacrylate (DiTMPTTA, $M_w$ = 438, viscosity = 1100 mPa.s (available as E140 from UCB Chemicals) and 3 parts by weight based on 100 parts by weight of release composition of UV photo initiator (available as DAROCUR 1173 from Ciba-Geigy, Basel, Switzerland)).

The release coating was applied to a non-woven backing where no film layer was present, however. The nonwoven was a heat embossed 50 g/m$^2$ spun bond polypropylene non-woven web (fiber thickness ca. 20 microns, available as DALTEX 1050 from Don & Low, Angus, Scotland) with a thermally bonded pattern of diamond-shaped spots having a width of ca. 6 mm. The total embossed area was 19 %.

A thicker layer of pressure-sensitive adhesive was applied to the side of the non-woven opposite the release composition as compared to Examples 1-5. More adhesive was applied as some adhesive penetrated into the non-woven because the film barrier was absent.

The tape construction is summarized in Table 1 and the properties of the adhesive tape in Table 2.

*Comparative Example 1*

Example 1 was repeated, with the exception that the organic compound employed as part of the release coating composition was hexanediol diacrylate (viscosity ca. 10 mPa·s) at 70 wt. % in combination with RC 715 (meth)acrylated silicone. The viscosity of the release composition at coating was ca. 38 mPa·s.

After the coating composition was applied and cured, only 0.5 g/m$^2$ of release coating could be measured on the surface of the non-woven web. The resulting roll of tape was unwound with difficulty and there was delamination of the fibers from the backing onto the pressure-sensitive adhesive. The backing was damaged and the adhesive surface was contaminated heavily with fibers.

*Comparative Example 2*

Comparative Example 1 was repeated with another low viscosity organic compound combined with RC 715. The organic compound employed was tripropyleneglycol diacrylate (TPGDA) having a viscosity of 15 mPa·s. The viscosity of the release coating composition was about 48 mPa·s.

The release composition penetrated largely into the non-woven web (as in Comparative Example 1), leaving little effective silicone on its surface.

As the resulting roll of tape was unwound, severe delamination of the backing occurred as in Comparative Example 1.

*Comparative Example 3*

Example 1 was repeated with the exception that instead of 30 wt % RC-715 silicone, 30 % of a commercially available mixture of UV-curable silicones was employed . The silicone mixture, designated commercially as TEGO RC 708, is a 70/30 weight:weight mixture of an acrylate-functionalized polydimethylsiloxane (RC-726, functionality 108:6 or 18) and MQ resin. MQ resin is known to have the effect of increasing the release force of release compositions employed. RC 708 is available as TEGO RC-708 from Th. Goldschmidt AG, Essen, Germany.

The mixture of silicones was combined with 70 wt % ditrimethylolpropane tetraacrylate (DMPTA, $M_w$ = 438, viscosity = 1100 mPa·s, available as E140 from UCB Chemicals), just as in Example 1.

The release force as measured by the Keil test was 433 cN/2.54 cm. Due to the presence of the MQ resin, the release force was found to be too high and the nonwoven backing was delaminated as the adhesive layer was peeled away.

*Comparative Example 4*

Comparative Example 4 was prepared using a (meth)acrylate-functional polydimethylsiloxane (functionality = 18) available as TEGO RC-726 from Th. Goldschmidt AG, Essen, Germany. This silicone has a low number of acrylate groups available for curing and was difficult to cure quickly under economically feasible line speeds and curing conditions on relative open webs exposed to trace oxygen. The resulting silicone release composition was not cured effectively and was transferred at least partially to the adhesive surface as the adhesive was separated from the release layer.
The adhesive surface of the tape did not have acceptable adhesion to polyethylene after it had been contaminated with uncured silicone.

*Comparative Example 5*

Comparative Example 5 employed a release composition comprising two silicone components commonly recommended by the suppliers of UV-curable silicones. No additional organic compound was present.
The release characteristics of this recommended composition are not suitable for use with highly aggressive pressure-sensitive adhesives commonly employed in the diaper industry.

*Comparative Example* 6

A standard silicone blend as recommended by the manufacturers as giving fast curing and low release properties was employed. The resulting performance after aging showed tape delamination.

*Comparative Example* 7

Example 1 was repeated with the exception that a (meth)acrylate-functional silicone having a high functionality (8.0) was employed. This silicone is commercially available as TEGO RC-706 from Th. Goldschmidt AG, Essen, Germany.
An excessively high separation or release force as measured by the Keil test was observed. Due to the high acrylic functionality of the silicone, less silicone was available to provide release.

*Comparative Example* 8

Example 1 was repeated, with the exception that a release composition having only 5 wt. % of RC 715 silicone was employed. Tests show that an effective total weight (2.5 $g/m^2$, see Table 1) of release coating composition was present on the surface of the fibrous non-woven layer after curing, but the overall silicone content was too low to provide effective release from the aggressive rubber/resin adhesive used. The measured value for the Keil test was 132 cN/2.54 cm.

*Comparative Examples* 9 *and* 10

Example 1 was repeated with the exception that the release composition contained 40 % RC 715 and 70 % RC 715, respectively, as summarized in Table 1.
Both show excessively high release values as measured by the Keil test as shown in Table 2.

*Comparative Example 11*

Example 1 was repeated with the exception that the organic compound employed was a polyethylene glycol 600 diacrylate, having a viscosity of ca. 50-100 mPa·s, available as SR 610 from Cray Valley Sartomer, Paris, France.
The viscosity of the release coating composition at coating was 134 mPa·s and thus it was absorbed excessively by the web before curing as indicated by a measured coating weight of 1.0 $g/m^2$. Insufficient release material was present on the surface of the fibrous web and thus the separation force as measured by the Keil test was excessively high (120 cN/2.54 cm).

*Table 1*

| Ex. | PSA (g/m²) | Silicone compound | | | Organic compound | | Release Comp. | |
|---|---|---|---|---|---|---|---|---|
| | | Type (wt%) | Funct. | Visc. (mP.s) | Type (wt%) | Visc. (mP.s) | Visc. (mP.s) | Meas. amount (g/m²) |
| 1 | 35 | RC 715 (30) | 13 | 150 | E 140 (70) | 1100 | 870 | 2.5 |
| 2 | 26 | RC 715 (20) | 13 | 150 | E 140 (80) | 1100 | 940 | 2.5 |
| 3 | 26 | RC 715 (10) | 13 | 150 | E 140 (90) | 1100 | 970 | 1.9 |
| 4 | 26 | RC 902 (30) | 14 | 350 | E 140 (70) | 1100 | 1077 | 2.5 |
| 5 | 26 | RC 715 (20) | 13 | 150 | E 810 (80) | 500 | 437 | 1.8 |
| 6 | 50 | RC 715 (30) | 13 | 150 | E 140 (70) | 1100 | 870 | 2.5 |
| C1 | 26 | RC 715 (30) | 13 | 150 | HDDA (70) | 10 | 38 | 0.5 |
| C2 | 26 | RC 715 (30) | 13 | 150 | TPGDA (70) | 15 | 48 | 0.5 |
| C3 | 26 | RC 708 (30) | N/A | 3080 | E 140 (70) | 1100 | 1610 | 2.0 |
| C4 | | RC 726 (30) | 18 | 1190 | E 140 (70) | 1100 | 1095 | 2.3 |
| C5 | 26 | RC 706 (70) | 8 | 150 | RC 711 (30) | 580 | 270 | 1.5 |
| C6 | 26 | RC 715 (70) | 13 | 150 | RC 711 (30) | 580 | 310 | 1.5 |
| C7 | 26 | RC 706 (30) | 8 | 150 | E 140 (70) | 1100 | 576 | 1.2 |
| C8 | 26 | RC 715 (5) | 13 | 150 | E 140 (95) | 1100 | 980 | 2.5 |
| C9 | 26 | RC 715 (40) | 13 | 150 | E 140 (60) | 1100 | 630 | 1.3 |
| C10 | 26 | RC 715 (70) | 13 | 150 | E 140 (30) | 1100 | 320 | 0.9 |
| C11 | 26 | RC 715 (30) | 13 | 150 | SR 610 (70) | 50-100 | 134 | 1.0 |

F = polymeric film;
NW = thermally embossed non-woven

*Table 2*

| Ex. | 90° Peel, N/2.54 cm | Static shear, min | Roll unwind force, cN/2.54 cm (initial) | Roll unwind force, cN/2.54 cm (aged) | 90° release (Keil test), cN/2.54 cm |
|---|---|---|---|---|---|
| 1 | 7.1 | 56 | 20 | 78 | 36 |
| 2 | 7.7 | 95 | 20 | 55 | 75 |
| 3 | 7.5 | 118 | 32 | 75 | 73 |
| 4 | 7.5 | 60 | 7 | 40 | 21 |
| 5 | 8.3 | 340 | 19 | 94 | 70 |
| 6 | 8.2 | 320 | 12 | 98 | 60 |
| C1 | — | — | * | * | * |
| C2 | — | — | * | * | * |
| C3 | 6.5 | 110 | 15 | 198 | 433* |
| C4 | 4.4 | 39 | 3** | 23 | 12** |
| C5 | 7.5 | 151 | 40 | * | 580 |
| C6 | 6.40 | 240 | 50 | * | 330 |
| C7 | — | — | — | — | 109 |
| C8 | 5.0 | 65 | 24 | 92 | 132 |
| C9 | --- | --- | --- | --- | 109 |
| C10 | --- | --- | --- | --- | 117 |
| C11 | 6.0 | 31 | 10 | 85 | 120 |

\* Tape delaminated on attempt to unwind roll. Fibers from the non-woven were transferred to adhesive surface.

\*\* Silicone coating was poorly cured

--- Not measured

## Claims

1. A method of making a release coated backing having a fibrous layer of woven fibers or of non-woven fibers of thermoplastic polymer, comprising the steps of coating a curable silicone release coating composition on the fibrous layer of the backing and curing the thus applied silicone release coating by exposing it to actinic radiation or heat, said curable silicone release coating composition comprising

   (i) a polydialkylsiloxane having acrylate and/or methacrylate groups and the ratio of the average number of dialkylsiloxane units to the average number of acrylate and methacrylate groups being between 10 and 15, and (ii) an organic compound free of silicon and comprising at least two reactive groups selected from the group consisting of an acrylate and a methacrylate group, said organic compound free of silicon having a viscosity of at least 500 mPa s at 25°C, and the weight ratio of said polydialkylsiloxane to said organic compound being between 8:92 and 35:65.

2. A method according to claim 1 wherein said curable silicone release coating composition further comprises a photo-initiator and said release coating is exposed to actinic radiation.

3. A method for making an adhesive coated tape comprising the steps of providing a release coated backing by the method defined in claim 1 and applying an adhesive layer to the side of the backing opposite to the side having the silicone release coating.

4. A release coating composition comprising:

   (i) a polydialkylsiloxane having acrylate and/or methacrylate groups and the ratio of the average number of dialkylsiloxane units to the average number of acrylate and methacrylate groups being between 10 and 15,
(ii) an organic compound free of silicon and comprising at least two reactive groups selected from the group consisting of an acrylate and a methacrylate group, said organic compound free of silicon having a viscosity of at least 500 mPa s at 25°C, and the weight ratio of said polydialkylsiloxane to said organic compound being between 8:92 and 35:65, and
(iii) optionally a photo-initiator.

5. A release coating obtained by curing a release coating comprising:

(i) a polydialkylsiloxane having acrylate and/or methacrylate groups and the ratio of the average number of dialkylsiloxane units to the average number of acrylate and methacrylate groups being between 10 and 15,
(ii) an organic compound free of silicon and comprising at least two reactive groups selected from the group consisting of an acrylate and a methacrylate group, said organic compound free of silicon having a viscosity of at least 500mPa s at 25°C, and the weight ratio of said polydialkylsiloxane to said organic compound being between 8:92 and 35:65, and
(iii) optionally a photo-initiator.

Fig. 1

Fig. 2

Fig. 3

Fig. 4

Fig. 5

Fig. 6